# NEW EUROPEAN PATENT SPECIFICATION

(11) **EP 3 503 116 B2**
(45) Date of publication and mention of the opposition decision: **28.01.2026**
(45) Mention of the grant of the patent: 01.04.2020
(21) Application number: 17209975.6
(22) Date of filing: 22.12.2017
(51) Int. Cl.: G16H 40/67

(54) **APPARATUS, METHOD AND SYSTEM FOR OBTAINING INFORMATION ON AN EMERGENCY SITUATION**
VORRICHTUNG, VERFAHREN UND SYSTEM ZUM ERHALTEN VON INFORMATIONEN ÜBER EINE NOTFALLSITUATION
APPAREIL, PROCÉDÉ ET SYSTÈME PERMETTANT D'OBTENIR DES INFORMATIONS SUR UNE SITUATION D'URGENCE

(43) Date of publication of application: 26.06.2019
(73) Proprietor: Corevas GmbH & Co. KG, 41516 Grevenbroich (DE)
(72) Inventor: Huhle, Viktor, 41516 Grevenbroich (DE); Huhle, Guenter, 41516 Grevenbroich (DE); Weinmann, Richard Josef, 69221 Dossenheim (DE); Petri, Carola, 41516 Grevenbroich (DE); Beck, Christoph, 6340 Baar (CH)
(74) Representative: Durm Patentanwälte PartG mbB

(56) References cited:
- WO-A1-2017/070323
- US-A1- 2012 222 129
- US-A1- 2015 056 946
- US-A1- 2017 293 730
- VIKRAM CHANDRASEKARAN ET AL: "Socio-technical aspects of remote media control for a NG9-1-1 system", MULTIMEDIA TOOLS AND APPLICATIONS., vol. 62, no. 3, 13 September 2011 (2011-09-13), US, pages 733 - 759, XP055484964, ISSN: 1380-7501, DOI: 10.1007/s11042-011-0875-1

## Description

The present invention relates to a method and a system for obtaining information on an emergency situation. Emergency situations involving injured people, e.g. heart attacks, strokes but also car or bike accidents, often occur in environments where no medical personnel is present. For instance, based on data from the German Resuscitation Register, annually 30 to 90 people out of 100,000 inhabitants experience a cardiac arrest. Less than 10 % survive an event that requires resuscitation. This means 250 deaths caused by cardiac arrest per day in Germany. When a cardiac arrest or another emergency situation occurs, timing and quality of first aid measures have a significant effect on the chances of survival and recovery.

Often, such situations are witnessed or discovered by bystanders without any medical experience. The usual and adequate reaction is to place an emergency call to an emergency control center (also referred to as emergency coordination center) which can organize help based on the information obtained from the bystander. The dispatcher in the emergency control center asks the caller about the location of the emergency situation and tries to obtain as much information as possible both on the injured person or injured persons and the circumstances and the surroundings. Based on this information, the dispatcher then organizes an adequate rescue operation.

For instance, not every bike accident requires the use of an emergency doctor in a helicopter whereas a single emergency doctor would be unable to cope with a mass casualty event resulting from a multiple car crash on an autobahn. Furthermore, the circumstances or surroundings may make it necessary that specially trained personnel or special tools are brought to the emergency situation. Still further, certain situations also make immediate first aid or resuscitation measures by the bystander necessary. In these cases, the dispatcher instructs the bystander to carry out the necessary and adequate tasks.

However, an emergency situation is always a stress situation and lays are quickly overwhelmed due to their lack of experience. It is known that in emergency situations with high stress levels lays can only recall very simple behavioral patterns, such as calling the national emergency number. If a necessity to act quickly arises, this puts additional pressure on all involved parties. On the one hand, the dispatcher knows that the bystander is in a stress situation and should not be put under too much pressure. One the other hand, a quick and adequate reaction is of utmost importance in a critical situation so that it is very relevant to obtain the necessary information on the emergency situation as fast as possible.

One option to better cope with the need to quickly obtain accurate information is the use of modern mobile communication devices. According to studies, more than 60 % of all emergency calls in Germany are set off from smartphones having the capability to capture and transmit digital images, videos and other sensor data. These data could be a life-saver in emergency situations since they allow obtaining objective information on the emergency situation without requiring to rely on the oral description of the bystander.

In this respect, DE 10 2013 008 133 A1 discloses a method and system to transmit a text-based emergency message using a mobile telecommunication device, a telecommunication network, a system, computer program and a computer program product. An emergency message is triggered by a telecommunication transmitting device and sent as a facsimile message to the emergency coordination center.

In Chandrasekaran et al., Socio-technical aspects of remote media control for a NG9-1-1 system, September 13, 2011, a VoIP based Next Generation 9-1-1 (NG9-1-1) system for remote media control is proposed. Specifically, a Session Initiation Protocol (SIP) is used in the implementation of the system using a mobile and a PC client. The proposed system on the mobile client accounts for less than 25% of CPU utilization even with video transmission. The average network utilization was about 10 and 72 kbps for audio and video, respectively.

US 2012/0222129 A1 relates to a system and method for secure mobile application download. Applications are downloaded to a mobile communicator and access to stored mobile applications is protected.

WO 2017/070323 A1 relates to an attentive assistant. The approach to providing communication assistance to an operator of a vehicle makes use of software having a first component executing on a personal device of the operator as well as a second component executing on a server in communication with the personal device.

US 2017/0293730 A1 relates to a remote-controlled medical assistance device. A system for treating a patient, including a computerized device with a display and a communication interface for communicating with remote mobile devices, one or more sensors for measuring medical parameters of a user is proposed. The sensors are controlled by the computerized device and transfer recorded measurements to the computerized device. An application executed on the computerized device allows a remote practitioner with a remote mobile device to communicate with the computerized device to control the sensors.

It is, however, usually not feasible to require a bystander to carry out a specific action like taking a photo and sending it via email with his mobile device due to the stress situation. Also, the use of a kind of emergency app on a smartphone is no practicable, since it is impossible to have such an app always preinstalled on all devices and since the bystander in the emergency situation will not have the time and concentration to use an app that he presumably has never used before.

Thus, currently no system exists that fully exploits the possibilities of modern mobile devices with respect to providing data and information on emergency situations. In view of this, it is the object of the present invention to provide a method and system for obtaining information on an emergency situation. The system should be simple to use for a bystander in a stress situation but nevertheless allow adequately and quickly assessing the emergency situation for a dispatcher in an emergency control center. All different data that could be of use for assessing the emergency situation and thereby assure a quick and adequate help in the emergency situation should be captured.

In aspects of the present invention this problem is solved by a method and a system as defined in the independent claims.

In yet further aspects, a corresponding computer program is provided which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed system, in particular as defined in the dependent claims and as disclosed herein.

The present invention is based on the idea that identification information of a mobile device is used to obtain remote access to the mobile device to remotely control the sensors and/or the actors of the mobile device. A bystander reports an emergency situation with a mobile device, e.g. by placing an emergency call (through an acoustic communication channel) or by sending a text message or by making use of an internet-based reporting mechanism with his smartphone. The mobile device is identified via identification information such as a telephone number, a MAC address, an IP address etc. Then, a deployment data packet is transmitted to the identified mobile device. Based on the deployment data packet, a control application is executed on the mobile device that allows obtaining remote access and that allows remotely controlling a sensor and/or an actor of the mobile device. Once this control application is executed, it is possible to communicate with the mobile device to remotely obtain information via the control sensor and/or actor. In other words, a data channel is opened. In particular, the dispatcher can access the functionalities of the device. Through this data channel, the dispatcher can communicate with the mobile device and access its sensors and/or actors.

As used herein an emergency situation particularly refers to any situation involving at least one injured person. Identification information is information that allows distinguishing one mobile device from another. In particular, a unique identification is advantageous. A deployment data packet is preferably understood as information that is transmitted from the apparatus to the mobile device. This deployment data packet forms the basis for executing a control application on the mobile device. In this respect, a deployment data packet may particularly correspond to any kind of digitally encoded information. A control application may particularly be a program to be run on a processor that controls at least one sensor or actor that is connected to said processor. The term remote, as used herein, relates to the access from a distance. In particular, remote access means that a dispatcher in an emergency control center can access a mobile device at the location of the emergency situation via mobile communication. It might be possible that the emergency control center is located in a different city or even in a different country than the emergency situation and that the remote access is performed over this distance. The communication of the apparatus with the mobile device is also preferably effected via a mobile communication network. In particular, a data network may be used. The controllable sensors/actors may particularly include a camera, a vital sign sensor, a position sensor, an acceleration sensor, a flash light and a loudspeaker. Obviously, it is preferred that multiple sensors/actors are controlled.

In comparison to previous approaches to cope with emergency situations, the present invention has the advantage that the bystander, i.e. the user of the mobile device, is not required to perform any specific control tasks with the mobile device. The control is carried out remotely by the dispatcher in the emergency control center. The person at the location of the emergency event only has to provide identification information of his mobile device. Then the sensors and/or actors of the mobile device are remotely controlled by trained personnel. Thus, in spite of the bystander possibly not being able to control his mobile device adequately due to the stress in the emergency situation, the data acquired by the different sensors of the mobile device can be provided to the emergency dispatcher to assess the situation. The pressure is taken away from the bystander. Trained personnel can remotely obtain the required information to coordinate a rescue operation. A higher efficiency in responding to emergency situations and coordinating adequate rescue operations can be obtained.

In a preferred embodiment, the application information includes at least one of: installer data for installing the control application on the mobile device and/or a download link to an internet server for providing the installer data; and an executable file for executing the control application on the mobile device and/or a download link to an internet server for providing the executable file. On the one hand, it is possible that the control application is installed on the mobile device. On the other hand, it is possible that no installation is required but that the control application is directly executed without installation. For both options, either a download link to the installer or the executable file can be provided as the application information in the deployment data packet, or the installer or executable file itself can be transmitted. The advantage of transmitting only a download link is that only a small amount of data has to be transmitted so that resources can be saved. By making use of an installable control application, the sensors of the mobile device can be accessed more efficiently. However, the transmission of an executable file, preferably running in a predefined framework, may allow the use of a platform-independent control application.

In a preferred embodiment, the transmission unit is configured to transmit the deployment data packet to the mobile device via an SMS, MMS, push message and/or instant message. The use of such predefined data transmission mechanisms allows an efficient implementation and provides a reliable communication. An immediate transmission of the deployment data packet can be assured.

In an embodiment, the transmission unit is configured to communicate with the mobile device to obtain device information of the mobile device being indicative of a device type and/or a version of the mobile device prior to transmitting the deployment data packet; and transmit the deployment data packet based on the obtained device information. In order to provide an adequate deployment data packet including application deployment information that is suitable for being used on the specific device, a prior communication of the apparatus with the mobile device may be carried out. Based on a device type and/or a version of the mobile device, the apparatus can provide an appropriate deployment data packet being suitable for being used on the respective device. Thereby, it can be assured that all different types and versions of mobile devices can be used in the system of the present invention. A wide compatibility and usability is obtained.

In yet another preferred embodiment, the ID-interface is configured to receive the identification information from an emergency control center for receiving an emergency situation report from a user of a mobile device reporting the emergency situation with the mobile device. In such an emergency control center, reports of emergency situations are centrally received, e.g. by means of phone calls, and rescue efforts and operations are coordinated. It may be decided by a human dispatcher or by an automatic routine whether the respective mobile device should be accessed remotely. In this case, the identification information can be provided to the apparatus via its ID-interface. This has the advantage that the course of events in the reporting of an emergency situation does not have to be modified. The bystander can report the emergency situation to the emergency control center, e.g. by placing an emergency phone call. The emergency control center communicates with the apparatus in the system of the present invention to remotely control the mobile device of the bystander. No additional burden is put on the bystander and it is assured that an adequate rescue operation is initiated.

In yet another embodiment, the transmission unit and/or the control unit are configured for encrypted communication with the mobile device, in particular end-to-end encrypted communication, and/or for authentication of the mobile device. It is important that an authentication of the mobile device is carried out to assure that an abuse of the system is impossible. If the mobile device is authenticated, it is clear which mobile device has reported the emergency situation and should therefore be remotely accessed. Additionally or alternatively, the use of an encrypted communication allows to assure that only the competent authority can access the possibly sensitive data of the mobile device that are obtained remotely. In this respect, an end-to-end encrypted communication particularly refers to an encryption from the mobile device to the dispatcher that eventually analyses the obtained data to assess the emergency situation. In other words, it might be possible that the apparatus in the system of the present invention only forwards encrypted data to the emergency control center. Data security and protection as well as compliance with respective laws is assured.

In another preferred embodiment, the control unit is configured to control a user interface of the mobile device via the control application to interact with a user of the mobile device and to provide instructions to the user on how to orient and/or position the mobile device, in particular the sensor and/or the actor of the mobile device, and/or on how to examine or resuscitate an injured person. It is also possible that the user of the mobile device, i.e. the bystander, is provided with instructions via the data channel. In particular, such instructions might include directions as to how to orient and/or position the mobile device to put the sensors or actors in an adequate position for observing the emergency situation. If the bystander calls the emergency control center via a regular phone call, he may obtain his instructions via this phone call. However, in an embodiment it is also possible that the instructions are provided via the apparatus and its control unit through the control application, i.e. through the data channel. This has the advantage that the phone call could be ended to save resources and to allow the user of the mobile device to focus his attention on the mobile device. An efficient reaction to the emergency situation can be assured.

The transmission unit is configured to: communicate with the mobile device to obtain a user consent information of a user of the mobile device indicating whether the user agrees to the control application being executed on the mobile device prior to transmitting the deployment data packet; and transmit the deployment data packet if the user consent information indicates that the user agrees to the control application being executed on the mobile device. It might be necessary to obtain a user consent prior to accessing functions of a mobile device, e.g. due to legal requirements. For this, a communication with the mobile device is carried out before transmitting the deployment data packet to obtain this user consent. Only if the user agrees to share the information of his mobile device and to allow access to his mobile device, the deployment data packet is transmitted. Thereby, it is assured that it is complied with corresponding legal requirements, if required.

In an embodiment, the control unit is configured to suppress a functionality of the mobile device while communicating with the mobile device. Thereby, resources of the mobile device are saved. By suppressing a functionality of the mobile device that is not related to the emergency situation, it becomes possible to save resources and thereby increase the lifetime of the mobile device. For instance, instant messaging services or regular e-mail pulls can be disabled during the emergency situation to reserve as many resources, in particular battery power, as possible for the emergency situation. This has the advantage that a maximum lifetime of the mobile device is assured by disabling all unnecessary functionalities.

In the method there are further comprised steps of receiving an emergency phone call placed by a user of a mobile device to report an emergency situation with the mobile device; and determining a telephone number of said mobile device as identification information. An emergency phone call is received and a telephone number is used as identification information. Thereby, the regular course of action can be maintained so that the person reporting the emergency situation can stay with accustomed processes. No additional pressure is placed on the bystander.

In the system the mobile device is a smartphone and the identification information includes a telephone number of the smartphone, the system comprising a user interface for an emergency dispatcher receiving an emergency phone call from a user of the smartphone calling to report an emergency situation with the smartphone, said user interface: being in communication with the control unit to provide to the emergency dispatcher information on a type of the mobile device and an availability of a specific sensor and/or actor; and providing control options for controlling the sensor and/or actor of the mobile device via the control unit and the control application. The user interface for the emergency dispatcher might particularly correspond to a web interface or the like that allows a comfortable and effective control of the remotely available functionalities of the mobile device. The dispatcher can quickly obtain a full image of the emergency situation and thereupon plan an adequate rescue operation.

These and other aspects of the invention will be apparent from and elucidated with reference to the embodiment(s) described hereinafter. In the following drawings
Figure 1 shows a schematic illustration of a system according to the present invention;
Figure 2 shows a schematic illustration of an apparatus of a system according to the present invention;
Figure 3 schematically illustrates a method of the present invention; and
Figure 4 shows a sequence diagram of the communication between the components of the system and the involved parties.

The present invention aims at better exploiting the possibilities of modern mobile electronic devices such as tablets, smartphones etc. in emergency situations. Currently, these communication devices are mainly used for placing an emergency call or making use of another type of communication to report the emergency situation to an emergency control center. Usually, the bystander, i.e. the person witnessing or discovering the emergency situation, calls a dispatcher in the emergency control center via a voice call to inform the rescuers of the situation.

However, lay first aiders who experience an emergency are often traumatized by such an event. Due to their lack of experience, they are often not able to provide the dispatcher with the necessary information so that the organization of the appropriate rescue operation could become difficult and time-consuming. In addition, oral descriptions are oftentimes not sufficiently accurate for the dispatcher to obtain a clear view of the situation.

According to the present invention it is proposed to improve this situation by making use of sensors and actors of a mobile device. The bystander of the emergency situation corresponding to the user of the mobile device does not need to control the mobile device by himself, e.g. by making use of a complicated and potentially unfamiliar emergency app, while being in a stress situation. The mobile device is controlled remotely and provides the data of its sensors to an emergency control center located a t a distance.

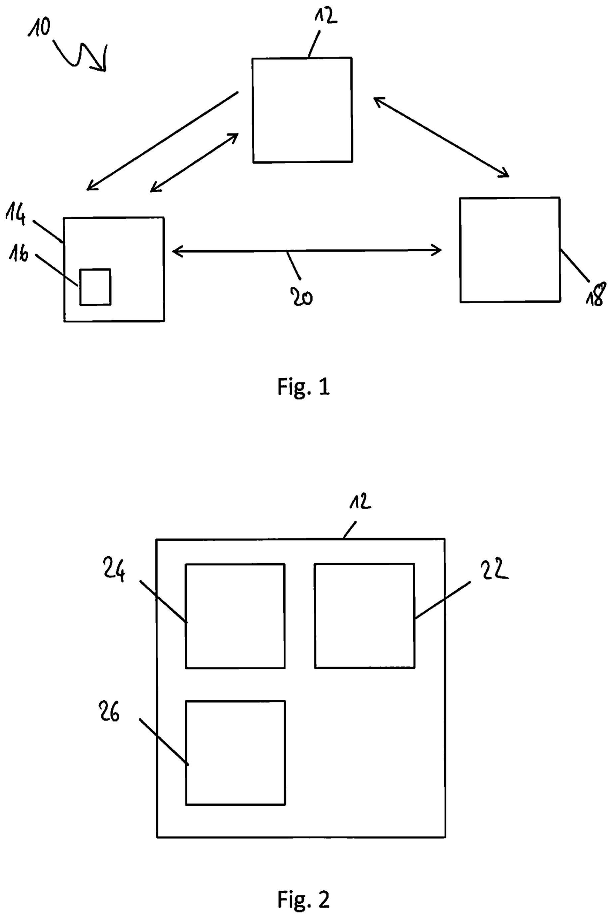

Figure 1 schematically illustrates a system 10 of the present invention. The system 10 includes an apparatus 12 for obtaining information on an emergency situation. In particular, the apparatus 12 may be implemented in the form of an internet server with interfaces to the GSM network and to the internet. Thus, the apparatus 12 may correspond to a software for being carried out on an internet server.

The system 10 further includes a mobile device 14 at a site of an emergency. The mobile device includes at least one sensor and/or actor 16 with which data on the emergency situation can be obtained or with which an interaction with the user can be carried out, respectively. The emergency situation is observed by means of the sensor and/or actor 16. The mobile device 14 may particularly correspond to a smartphone. The emergency situation is reported via said mobile device 14 by a bystander, i.e. a person using said mobile device 14. For this, in particular an acoustic channel to an emergency control center is established.

The system 10 is in communication with an emergency control center 18 with an emergency dispatcher, who is trained to coordinate a rescue operation. In the emergency control center, there is usually provided a user interface for the dispatcher to communicate and interact with the apparatus 12.

Usually, an emergency phone call is placed by the bystander via a voice channel 20. The dispatcher can then provide the apparatus 12 with identification information of the mobile device 14. In particular, the telephone number of a smartphone can represent the identification information. Based on this identification information, the apparatus 12 transmits a deployment data packet to the mobile device 14. This deployment data packet has the effect that a control application is executed on the mobile device 14. Via this control application, it is possible to remotely access the mobile device 14 from the apparatus 12 and to control at least one sensor or actor of the mobile device 14. By accessing a sensor and/or an actor of the mobile device 14, the dispatcher in the emergency control center 18 can obtain direct data from the site of the emergency situation.

Figure 2 shows a schematic illustration of the apparatus 12. The apparatus 12 includes an ID-interface 22 which is configured to communicate with the emergency control center for receiving the identification information of the mobile device. This ID-interface 22 can, e.g., correspond to an internet interface. Further, the apparatus 12 includes a transmission unit 24 which is configured to transmit to the mobile device a deployment data packet for executing a control application. This transmission unit can correspond to an interface to a mobile communication network. In addition, the apparatus 12 includes a control unit 26 via which a data channel to the mobile device can be established to control the sensor and/or the actor of the mobile device. The control unit can correspond to a processor or a corresponding computer program. It is to be understood that the different units of the apparatus 12 may partly or completely be implemented in hard- and/or software.

In particular, the apparatus 12 may be configured to obtain information from a smartphone, tablet, tablet PC, personal digital assistant (PDA), or smartwatch etc. Such mobile devices usually include a plurality of relevant sensors and actors and are able to share data in real-time via data channels by streaming or making use of video telephony.

Thus, the control unit may be configured to control (or to allow control of) at least one of a camera, a position sensor, a display, a loudspeaker, a vital sign sensor, a flashlight and an acceleration sensor of the mobile device. Based on the data of the camera, visual information can be obtained. The position sensor can be used to obtain the exact location of the situation and the injured person. The display and the loudspeaker of the mobile device can be controlled to guide and direct the bystander, for instance if resuscitation actions are required. Data of a vital sign sensor or an acceleration sensor can be directly used to assess the situation of an injured person. A flashlight can be used to eliminate the injured person and the surroundings.

The ID-interface 22 may particularly receive identification information in the form of a telephone number of a smartphone corresponding to the mobile device with which the emergency situation is reported via an emergency phone call. Then, the establishment of a data channel and a communication between the mobile device 14 and the apparatus 12 may be initiated by the dispatcher in the emergency control center. For the transmission of the identification information, a preinstalled software on a computer in the emergency control center, a web interface or another secure channel preferably including individual authentication can be used. The remote control of the mobile device 14 is initiated from the emergency control center by submitting the telephone number of a mobile device to the apparatus 12. Based thereupon, the apparatus 12 initiates the remote access by sending the deployment data packet corresponding to a request to access sensors and/or actors to the mobile device. Upon receiving the data deployment packet, the mobile device 14 executes a control application.

The necessary data for executing the control application on the mobile device may either be directly transmitted via the deployment data packet. In this case, the deployment data packet can include an executable file or an installer file. Alternatively, the transmission unit 24 of the apparatus 12 may be configured to transmit a download link via which the executable file or the installer file can then be provided for the mobile device to download in a subsequent step. Preferably, the transmission unit transmits a download link via an SMS or other message.

The remote deployment of the control application may, for instance, be executed via a ROM package, in the form of an app from an app store, in the form of a verified app from other sources, in the form of an instant app or in the form of an HTML 5 package.

Once the data channel between the control application on the mobile device and the apparatus or the emergency control center, respectively, is established, it is possible for the dispatcher to control a sensor and/or an actor of the mobile device. Preferably, the apparatus 12 is in the form of an internet server that can be accessed by the dispatcher, e.g. through a corresponding web interface.

The communication between the mobile device and the apparatus or the emergency control center, respectively, is usually encrypted in order to protect the exchanged data. For this, an authentication of the mobile device can be carried out to assure the identity of the mobile device and its user. Then, an end-to-end encryption can be established. In particular, it is possible that the apparatus does not have access to the content of the data obtained from the mobile device but only forwards encrypted data to the dispatcher to decrypt it. Thereby, it is possible to comply with data protection regulations since the data that potentially includes medical information on an injured person, can only be seen by the authorized personnel in the emergency control center.

Prior to opening the data channel via transmitting the deployment data packet it is possible that the apparatus communicates with the mobile device to obtain device information from the mobile device. This device information describes the device type and/or a version of the mobile device. Thereby, it becomes possible to provide a device-specific deployment data packet that is suitable for the specific mobile device by which the emergency situation is reported. Depending on the device type a specific software is provided and a device-specific control application is executed on the mobile device.

In order to comply with data protection regulations, it may be required that prior to accessing the functions of the mobile device, the user's consent to share this data and to allow this access is obtained. The user is asked whether he agrees to a remote control of his mobile device. For this, a corresponding user consent information indicating whether or not the user agrees to have functions of his mobile device controlled is obtained. Only if the user agrees to this control, the deployment data packet is transmitted to the mobile device.

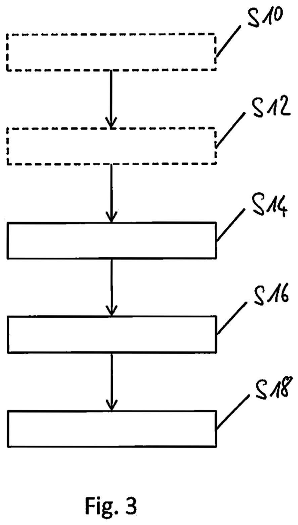

In Figure 3 a method of the present invention is illustrated. The method can particularly be implemented in the form of a computer program running on a server with an internet connection. The method includes steps of receiving S10 an emergency phone call, determining S12 a telephone number of the mobile device with which the emergency phone call is placed, receiving S14 identification information of the mobile device at an apparatus, transmitting S16 a deployment data packet to the mobile device, and communicating S18 with the mobile device by means of a data channel to obtain therefrom information on the emergency situation. It is to be understood that the steps of receiving S10 and determining S12 are optional and not necessary as indicated by the dashed outlines. It is possible that alternatively another report of the emergency situation is received and another type of identification information is determined.

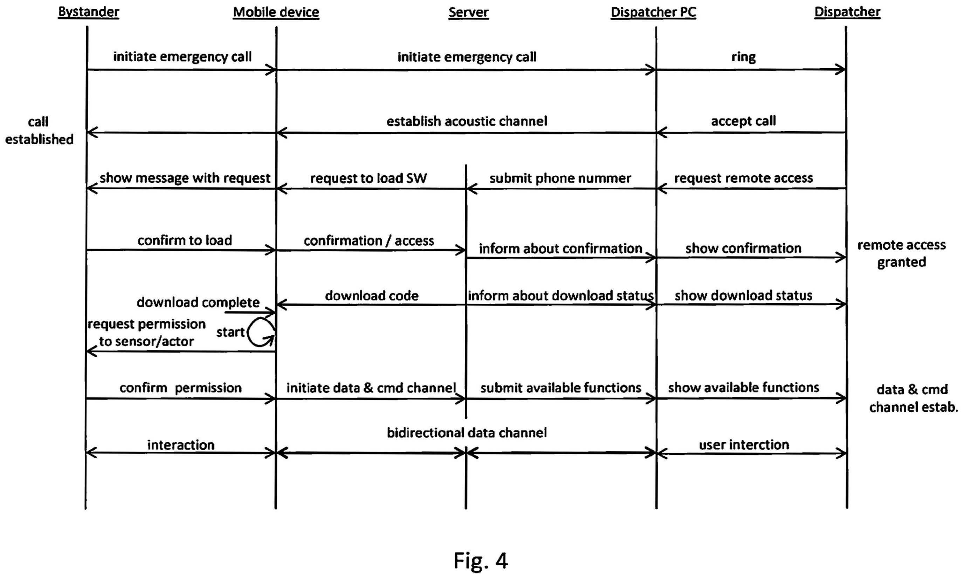

Figure 4 schematically illustrates the sequence of actions in an embodiment of the present invention. At first, an acoustic communication is initiated via a mobile phone. Then, the control application is downloaded to the mobile device. The bystander is asked for his consent to download the software. The dispatcher is informed that the user agrees to have the software installed on his mobile device. Then, the data channel is established to allow the dispatcher in the emergency control center to remotely control at least one sensor or actor on the mobile device.

While the invention has been illustrated and described in detail in the drawings and foregoing description, such illustration and description are to be considered illustrative or exemplary and not restrictive; the invention is not limited to the disclosed embodiments. Other variations of the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. System (10) for obtaining information on an emergency situation, comprising a mobile device (14) for reporting an emergency situation by placing an emergency call through an acoustic communication channel, said mobile device being a smartphone, and an apparatus (12) comprising:
an ID-interface (22) for receiving an identification information of the mobile device (14), said identification information including a telephone number of the smartphone;
a transmission unit (24) for transmitting a deployment data packet to the mobile device identified by the identification information, said deployment data packet including application deployment information for executing on the mobile device a control application for providing remote access to the mobile device to control a sensor (16) of the mobile device; and
a control unit (26) for communicating with the mobile device to obtain information on the emergency situation by obtaining remote access so that an emergency dispatcher can access functionalities of the mobile device and remotely control the sensor via the control application,
wherein the application deployment information includes at least one of: installer data for installing the control application on the mobile device (14) and/or a download link to an internet server for providing the installer data; and an executable file for executing the control application on the mobile device and/or a download link to an internet server for providing the executable file; and
wherein the transmission unit is configured to communicate with the mobile device (14) to obtain a user consent information of a user of the mobile device indicating whether the user agrees to the control application being executed on the mobile device prior to transmitting the deployment data packet; and transmit the deployment data packet if the user consent information indicates that the user agrees to the control application being executed on the mobile device;
the system further comprising a user interface for the emergency dispatcher receiving the emergency call from the user of the mobile device, said user interface being in communication with the control unit (26) to provide to the emergency dispatcher information on a type of the mobile device and an availability of a specific sensor (16), and said user interface providing control options for controlling the sensor of the mobile device via the control unit and the control application.

2. System (10) as claimed in claim 1, wherein the transmission unit is configured to transmit the deployment data packet to the mobile device (14) via an SMS, MMS, push message and/or instant message.

3. System (10) as claimed in any one of the preceding claims, wherein the transmission unit is configured to
communicate with the mobile device to obtain device information of the mobile device being indicative of a device type and/or a version of the mobile device prior to transmitting the deployment data packet; and
transmit the deployment data packet based on the obtained device information.

4. System (10) as claimed in any one of the preceding claims, wherein the ID-interface (22) is configured to receive identification information including a telephone number of a smartphone via which an emergency phone call is placed to report the emergency situation.

5. System (10) as claimed in any one of the preceding claims, wherein the ID-interface (22) is configured to receive the identification information from an emergency control center (18) for receiving an emergency situation report from a user of a mobile device (14) reporting the emergency situation with the mobile device.

6. System (10) as claimed in any one of the preceding claims, wherein the transmission unit and/or the control unit (26) are configured for encrypted communication with the mobile device (14), in particular end-to-end encrypted communication, and/or for authentication of the mobile device.

7. System (10) as claimed in any one of the preceding claims, wherein the control unit (26) is configured to control a user interface of the mobile device (14) via the control application to interact with a user of the mobile device and to provide instructions to the user on how to orient and/or position the mobile device, in particular the sensor (16) of the mobile device, and/or on how to examine or resuscitate an injured person.

8. System (10) as claimed in any one of the preceding claims, wherein the control unit (26) is configured to suppress a functionality of the mobile device (14) while communicating with the mobile device to safe resources of the mobile device.

9. Method for obtaining information on an emergency situation, comprising the steps:
receiving (S14) an identification information of a mobile device (14) via which the emergency situation is reported by placing an emergency call through an acoustic communication channel, said mobile device being a smartphone and said identification information including a telephone number of the smartphone;
transmitting (S16) a deployment data packet to the mobile device identified by the identification information, said deployment data packet including application deployment information for executing on the mobile device a control application for providing remote access to the mobile device to control a sensor (16) of the mobile device; and
communicating (S18) with the mobile device to obtain information on the emergency situation by obtaining remote access so that an emergency dispatcher can access functionalities of the mobile device and remotely control the sensor via the control application,
wherein the application deployment information includes at least one of: installer data for installing the control application on the mobile device **(14)** and/or a download link to an internet server for providing the installer data; and an executable file for executing the control application on the mobile device and/or a download link to an internet server for providing the executable file;
further comprising steps of communicating with the mobile device (14) to obtain a user consent information of a user of the mobile device indicating whether the user agrees to the control application being executed on the mobile device prior to transmitting the deployment data packet; and transmitting the deployment data packet if the user consent information indicates that the user agrees to the control application being executed on the mobile device; and
further comprising steps of providing to the emergency dispatcher information on a type of the mobile device and an availability of a specific sensor (16) via a user interface and providing, via said user interface, control options for controlling the sensor of the mobile device via the control application.

10. Computer program comprising program code means for causing a computer to carry out the steps of the method as claimed in claim 9 when said computer program is carried out on the computer.

## Patentansprüche

1. System (10) zum Erfassen von Informationen über eine Notfallsituation mit einem Mobilgerät (14) zum Melden einer Notfallsituation, indem ein Notruf durch einen akustischen Kommunikationskanal abgesetzt wird, wobei das Mobilgerät ein Smartphone ist, und eine Vorrichtung (12), umfassend:
eine ID-Schnittstelle (22) zum Empfangen einer Identifizierungsinformation des Mobilgeräts (14), wobei die Identifizierungsinformation eine Telefonnummer des Smartphones umfasst;
eine Übertragungseinheit (24) zum Übertragen eines Bereitstellungsdatenpakets an das durch die Identifizierungsinformation identifizierte Mobilgerät, wobei das Bereitstellungsdatenpaket Anwendungsbereitstellungsinformationen umfasst zum Ausführen einer Steuerungsanwendung auf dem Mobilgerät zum Bereitstellen eines Fernzugriffs auf das Mobilgerät zum Steuern eines Sensors (16) des Mobilgeräts; und
eine Steuerungseinheit (26) zum Kommunizieren mit dem Mobilgerät, um Informationen über die Notfallsituation zu erhalten durch das Erlangen von Fernzugriff, so dass ein Notrufverteiler auf Funktionalitäten des Mobilgeräts zugreifen und über die Steuerungsanwendung den Sensor fernsteuern kann,
wobei die Anwendungsbereitstellungsinformationen mindestens eines der folgenden Elemente umfassen: Installationsdaten zum Installieren der Steuerungsanwendung auf dem Mobilgerät (14) und/oder einen Downloadlink zu einem Internetserver zum Bereitstellen der Installationsdaten; und eine ausführbare Datei zum Ausführen der Steuerungsanwendung auf dem Mobilgerät und/oder einen Downloadlink zu einem Internetserver zum Bereitstellen der ausführbaren Datei; und
wobei die Übertragungseinheit dazu ausgebildet ist, mit dem Mobilgerät (14) zu kommunizieren, um vor dem Übertragen des Bereitstellungsdatenpakets eine Benutzerzustimmungsinformation eines Benutzers des Mobilgeräts zu erhalten, die anzeigt, ob der Benutzer einer Ausführung der Steuerungsanwendung auf dem Mobilgerät zustimmt; und das Bereitstellungsdatenpaket zu übertragen, wenn die Benutzerzustimmungsinformation anzeigt, dass der Benutzer einer Ausführung der Steuerungsanwendung auf dem Mobilgerät zustimmt;
das System ferner eine Benutzerschnittstelle für den Notrufverteiler umfasst, der den Notruf von einem Benutzer des Mobilgeräts empfängt, wobei die Benutzerschnittstelle mit der Steuerungseinheit (26) in Kommunikationsverbindung steht, um dem Notrufverteiler Informationen über einen Typ des Mobilgeräts und eine Verfügbarkeit eines bestimmten Sensors (16) zur Verfügung zu stellen, und die Benutzerschnittstelle Steuerungsoptionen zum Steuern des Sensors des Mobilgeräts über die Steuerungseinheit und die Steuerungsanwendung bereitstellt.

2. System (10) nach Anspruch 1, wobei die Übertragungseinheit dazu ausgebildet ist, das Bereitstellungsdatenpaket über eine SMS, MMS, Push-Nachricht und/oder Sofortnachricht an das Mobilgerät (14) zu übertragen.

3. System (10) nach einem der vorstehenden Ansprüche, wobei die Übertragungseinheit dazu ausgebildet ist,
mit dem Mobilgerät zu kommunizieren, um vor dem Übertragen des Bereitstellungsdatenpakets Geräteinformationen des Mobilgeräts zu erhalten, die auf einen Gerätetyp und/oder eine Version des Mobilgeräts hinweisen; und
das Bereitstellungsdatenpaket basierend auf den erhaltenen Geräteinformationen zu übertragen.

4. System (10) nach einem der vorstehenden Ansprüche, wobei die ID-Schnittstelle (22) dazu ausgebildet ist, Identifizierungsinformationen mit einer Telefonnummer eines Smartphones, über das ein Notruf abgesetzt wird, um die Notfallsituation zu melden, zu empfangen.

5. System (10) nach einem der vorstehenden Ansprüche, wobei die ID-Schnittstelle (22) dazu ausgebildet ist, die Identifizierungsinformation von einer Notfallzentrale (18) zum Empfangen eines Notfallsituationsberichts von einem Benutzer eines Mobilgeräts (14) zu empfangen, der die Notfallsituation mit dem Mobilgerät meldet.

6. System (10) nach einem der vorstehenden Ansprüche, wobei die Übertragungseinheit und/oder die Steuerungseinheit (26) für eine verschlüsselte Kommunikation mit dem Mobilgerät (14), insbesondere eine Endezu-Ende verschlüsselte Kommunikation, und/oder zur Authentifizierung des Mobilgeräts ausgebildet sind.

7. System (10) nach einem der vorstehenden Ansprüche, wobei die Steuerungseinheit (26) dazu ausgebildet ist, eine Benutzerschnittstelle des Mobilgeräts (14) über die Steuerungsanwendung zu steuern, um mit einem Benutzer des Mobilgeräts zu interagieren und um dem Benutzer Anweisungen zuzuleiten, wie das Mobilgerät, insbesondere der Sensor (16) des Mobilgeräts, auszurichten und/oder zu positionieren ist und/oder wie eine verletzte Person zu untersuchen oder wiederzubeleben ist.

8. System (10) nach einem der vorstehenden Ansprüche, wobei die Steuerungseinheit (26) dazu ausgebildet ist, eine Funktionalität des Mobilgeräts (14) während der Kommunikation mit dem Mobilgerät zu unterdrücken, um Ressourcen des Mobilgeräts zu schonen.

9. Verfahren zum Erfassen von Informationen über eine Notfallsituation, umfassend die Schritte:
Empfangen (S14) einer Identifizierungsinformation eines Mobilgeräts (14), über das die Notfallsituation gemeldet wird, indem ein Notruf durch einen akustischen Kommunikationskanal abgesetzt wird, wobei das Mobilgerät ein Smartphone ist und die Identifizierungsinformation eine Telefonnummer des Mobilgeräts umfasst;
Übertragen (S16) eines Bereitstellungsdatenpakets an das durch die Identifizierungsinformation identifizierte Mobilgerät, wobei das Bereitstellungsdatenpaket Anwendungsbereitstellungsinformationen umfasst zum Ausführen einer Steuerungsanwendung auf dem Mobilgerät zum Bereitstellen eines Fernzugriffs auf das Mobilgerät zum Steuern eines Sensors (16) des Mobilgeräts; und
Kommunizieren (S18) mit dem Mobilgerät, um Informationen über die Notfallsituation zu erhalten, indem Fernzugriff erlangt wird, so dass ein Notrufverteiler auf Funktionalitäten des Mobilgeräts zugreifen und über die Steuerungsanwendung den Sensor fernsteuern kann,
wobei die Anwendungsbereitstellungsinformationen mindestens eines der folgenden Elemente umfassen: Installationsdaten zum Installieren der Steuerungsanwendung auf dem Mobilgerät (14) und/oder einen Downloadlink zu einem Internetserver zum Bereitstellen der Installationsdaten; und eine ausführbare Datei zum Ausführen der Steuerungsanwendung auf dem Mobilgerät und/oder einen Downloadlink zu einem Internetserver zum Bereitstellen der ausführbaren Datei;
ferner umfassend die Schritte des Kommunizierens mit dem Mobilgerät (14), um vor dem Übertragen des Bereitstellungsdatenpakets eine Benutzerzustimmungsinformation eines Benutzers des Mobilgeräts zu erhalten, die anzeigt, ob der Benutzer einer Ausführung der Steuerungsanwendung auf dem Mobilgerät zustimmt; und des Übertragens des Bereitstellungsdatenpakets, wenn die Benutzerzustimmungsinformation anzeigt, dass der Benutzer einer Ausführung der Steuerungsanwendung auf dem Mobilgerät zustimmt; und
ferner umfassend die Schritte des Bereitstellens von Informationen an den Notrufverteiler über einen Typ des Mobilgeräts und eine Verfügbarkeit eines bestimmten Sensors (16) über eine Benutzerschnittstelle und des Bereitstellens von Steuerungsoptionen über die Schnittstelle zum Steuern des Sensors des Mobilgeräts über die Steuerungsanwendung.

10. Computerprogramm, umfassend einen Programmcode, um einen Computer zu veranlassen, die Schritte des Verfahrens nach Anspruch 9 auszuführen, wenn das Computerprogramm auf dem Computer ausgeführt wird.

## Revendications

1. Système (10) destiné à l'obtention d'informations relatives à une situation d'urgence, comprenant un dispositif mobile (14) destiné à signaler une situation d'urgence par l'établissement d'un appel d'urgence au travers d'un canal de communication acoustique, ledit dispositif mobile étant un téléphone multifonction, et un appareil (12), comprenant :
une interface ID (22) destinée à la réception d'informations d'identification du dispositif mobile (14), lesdites informations d'identification comprenant un numéro de téléphone du téléphone multifonction ;
une unité de transmission (24) destinée à la transmission d'un paquet de données de déploiement au dispositif mobile identifié par les informations d'identification, ledit paquet de données de déploiement comprenant des informations de déploiement d'application destinées à l'exécution, sur le dispositif mobile, d'une application de commande destinée à la fourniture d'un accès à distance au dispositif mobile afin de commander un capteur (16) du dispositif mobile ; et
une unité de commande (26) destinée à communiquer avec le dispositif mobile afin d'obtenir des informations relatives à la situation d'urgence par l'obtention d'un accès à distance, de sorte qu'un répartiteur d'urgences puisse accéder à des fonctionnalités du dispositif mobile et commander à distance le capteur par l'intermédiaire de l'application de commande,
dans lequel les informations de déploiement d'application comprennent au moins un élément parmi :
des données d'installation destinées à l'installation de l'application de commande sur le dispositif mobile (14) et/ou un lien de téléchargement vers un serveur Internet destiné à la fourniture des données d'installation ; et un fichier exécutable destiné à l'exécution de l'application de commande sur le dispositif mobile et/ou un lien de téléchargement vers un serveur Internet destiné à la fourniture du fichier exécutable ; et
dans lequel l'unité de transmission est configurée de façon à communiquer avec le dispositif mobile (14) afin d'obtenir, avant la transmission du paquet de données de déploiement, des informations de consentement d'utilisateur d'un utilisateur du dispositif mobile indiquant si l'utilisateur accepte que l'application de commande soit exécutée sur le dispositif mobile, et à transmettre le paquet de données de déploiement si les informations de consentement d'utilisateur indiquent que l'utilisateur accepte que l'application de commande soit exécutée sur le dispositif mobile ;
le système comprenant en outre une interface utilisateur destinée au répartiteur d'urgences recevant l'appel d'urgence de l'utilisateur du dispositif mobile, ladite interface utilisateur étant en communication avec l'unité de commande (26) afin de fournir au répartiteur d'urgences des informations relatives à un type du dispositif mobile et à une disponibilité d'un capteur spécifique (16), et ladite interface utilisateur fournissant des options de commande destinées à la commande du capteur du dispositif mobile par l'intermédiaire de l'unité de commande et de l'application de commande.

2. Système (10) selon la revendication 1, dans lequel l'unité de transmission est configurée de façon à transmettre le paquet de données de déploiement au dispositif mobile (14) par l'intermédiaire d'un SMS, d'un MMS, d'un message poussé et/ou d'un message instantané.

3. Système (10) selon l'une quelconque des revendications précédentes, dans lequel l'unité de transmission est configurée de façon à communiquer avec le dispositif mobile afin d'obtenir, avant la transmission du paquet de données de déploiement, des informations de dispositif du dispositif mobile indicatives d'un type de dispositif et/ou d'une version du dispositif mobile, et à transmettre le paquet de données de déploiement en fonction des informations de dispositif obtenues.

4. Système (10) selon l'une quelconque des revendications précédentes, dans lequel l'interface ID (22) est configurée de façon à recevoir des informations d'identification comprenant un numéro de téléphone d'un téléphone multifonction par l'intermédiaire duquel un appel téléphonique d'urgence est établi afin de signaler la situation d'urgence.

5. Système (10) selon l'une quelconque des revendications précédentes, dans lequel l'interface ID (22) est configurée de façon à recevoir les informations d'identification à partir d'un centre de contrôle d'urgence (18) destiné à la réception d'un signalement de situation d'urgence provenant d'un utilisateur d'un dispositif mobile (14).

6. Système (10) selon l'une quelconque des revendications précédentes, dans lequel l'unité de transmission et/ou l'unité de commande (26) sont configurées pour une communication chiffrée avec le dispositif mobile (14), plus particulièrement une communication chiffrée de bout en bout, et/ou pour une authentification du dispositif mobile.

7. Système (10) selon l'une quelconque des revendications précédentes, dans lequel l'unité de commande (26) est configurée de façon à commander une interface utilisateur du dispositif mobile (14) par l'intermédiaire de l'application de commande afin d'interagir avec un utilisateur du dispositif mobile et de fournir à l'utilisateur des instructions indiquant comment orienter et/ou positionner le dispositif mobile, plus particulièrement le capteur (16) du dispositif mobile, et/ou indiquant comment examiner ou ranimer une personne blessée.

8. Système (10) selon l'une quelconque des revendications précédentes, dans lequel l'unité de commande (26) est configurée de façon à supprimer une fonctionnalité du dispositif mobile (14) pendant la communication avec le dispositif mobile afin de préserver des ressources du dispositif mobile.

9. Procédé d'obtention d'informations relatives à une situation d'urgence, comprenant les étapes suivantes :
la réception **(S14)** d'informations d'identification d'un dispositif mobile (14) par l'intermédiaire duquel la situation d'urgence est signalée par l'établissement d'un appel d'urgence au travers d'un canal de communication acoustique, ledit dispositif mobile étant un téléphone multifonction et lesdites informations d'identification comprenant un numéro de téléphone du téléphone multifonction ;
la transmission (S16) d'un paquet de données de déploiement au dispositif mobile identifié par les informations d'identification, ledit paquet de données de déploiement comprenant des informations de déploiement d'application destinées à l'exécution, sur le dispositif mobile, d'une application de commande destinée à la fourniture d'un accès à distance au dispositif mobile afin de commander un capteur (16) du dispositif mobile ; et
la communication (S18) avec le dispositif mobile afin d'obtenir des informations relatives à la situation d'urgence par l'obtention d'un accès à distance, de sorte qu'un répartiteur d'urgences puisse accéder à des fonctionnalités du dispositif mobile et commander à distance le capteur par l'intermédiaire de l'application de commande,
dans lequel les informations de déploiement d'application comprennent au moins un élément parmi :
des données d'installation destinées à l'installation de l'application de commande sur le dispositif mobile (14) et/ou un lien de téléchargement vers un serveur Internet destiné à la fourniture des données d'installation;
et un fichier exécutable destiné à l'exécution de l'application de commande sur le dispositif mobile et/ou un lien de téléchargement vers un serveur Internet destiné à la fourniture du fichier exécutable ;
le procédé comprenant en outre les étapes consistant à communiquer avec le dispositif mobile (14) afin d'obtenir, avant la transmission du paquet de données de déploiement, des informations de consentement d'utilisateur indiquant si l'utilisateur accepte que l'application de commande soit exécutée sur le dispositif mobile, et à transmettre le paquet de données de déploiement si les informations de consentement d'utilisateur indiquent que l'utilisateur accepte que l'application de commande soit exécutée sur le dispositif mobile ;
le procédé comprenant en outre les étapes consistant à fournir, à un répartiteur d'urgences, par l'intermédiaire d'une interface utilisateur, des informations relatives à un type du dispositif mobile et à une disponibilité d'un capteur spécifique (16), et à fournir, par ladite interface utilisateur, des options de commande destinées à la commande du capteur du dispositif mobile par l'intermédiaire de l'application de commande.

10. Programme informatique comprenant un moyen de code de programme destiné à amener un ordinateur à exécuter les étapes du procédé selon la revendication 9 lorsque ledit programme informatique est exécuté sur l'ordinateur.
